(19) Europäisches Patentamt
European Patent Office
Office européen des brevets

(11) Veröffentlichungsnummer: **0 501 205 B1**

(12) **EUROPÄISCHE PATENTSCHRIFT**

(45) Veröffentlichungstag der Patentschrift: **24.05.95**

(51) Int. Cl.$^{6}$: **A61K 31/19**, A61K 31/21

(21) Anmeldenummer: **92102061.6**

(22) Anmeldetag: **07.02.92**

Die Akte enthält technische Angaben, die nach dem Eingang der Anmeldung eingereicht wurden und die nicht in dieser Patentschrift enthalten sind.

# (54) Antiphlogistisches Mittel.

(30) Priorität: **26.02.91 DE 4106026**

(43) Veröffentlichungstag der Anmeldung:
**02.09.92 Patentblatt 92/36**

(45) Bekanntmachung des Hinweises auf die Patenterteilung:
**24.05.95 Patentblatt 95/21**

(84) Benannte Vertragsstaaten:
**AT BE CH DE DK ES FR GB GR IT LI LU NL PT SE**

(56) Entgegenhaltungen:
**WO-A-90/04968**

**INT. ARCH. ALLERGY APPL. IMMUNOL., Bd. 94, Nr. 1-4, 1991, Seiten 262-265; W.DORSCH ET AL: 'New Antiasthmatic Drugs from Traditional Medicine?'**

**JOURNAL OF NATURAL PRODUCTS, Bd. 52, Nr. 4, 1989, Seiten 762-768; M. NISHIZAWA ET AL: 'Anti-Aids Agents'**

**JOURNAL OF NATURAL PRODUCTS, Bd. 50, Nr. 3, 1987, Seiten 392-399; YOSHIYUKI KIMURA ET AL.: 'Studies on the activities of Tannins and related compounds'**

**BIOMED. BIOCHIM. ACTA, Bd. 45, Nr. 10, 1986, Seiten 1267-1275; J.SLAPKE ET AL.: 'Pure enzymes in a test hierarchy for antibronchoconstrictory lipoxygenase inhibitors'**

(73) Patentinhaber: **Plantamed Arzneimittel GmbH**
**Kerschensteiner Strasse 11-15**
**D-92318 Neumarkt (DE)**

(72) Erfinder: **Wagner, Hildebert, Prof. Dr. Dr., Inst. für pharm.**
**Biologie der Universität München,**
**Karlstrasse 29**
**W-8000 München 2 (DE)**
Erfinder: **Dorsch, Walter, Prof. Dr.**
**Schönstrasse 71B**
**W-8000 München 90 (DE)**

(74) Vertreter: **Sandmair, Kurt, Dr. Dr.**
**Patentanwälte**
**Schwabe, Sandmair, Marx**
**Stuntzstrasse 16**
**D-81677 München (DE)**

EP 0 501 205 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung bestimmter Gallussäureverbindungen zur Herstellung einer pharmazeutischen Zubereitung zur Behandlung entzündlicher Erkrankungen.

Die pharmazeutischen Zubereitungen sind zur Behandlung von entzündlichen Erkrankungen sämtlicher Organe, insbesondere von Erkrankungen des rheumatischen Formenkreises, allergischen und immunologischen Erkrankungen, Asthmabronchiale und anderen Lungenerkrankungen wie obstruktive Bronchitis und entzündliche Hauterkrankungen brauchbar (Antiphlogistika).

Unter dem Begriff der Entzündung, wie er hier zu verstehen ist, wird allgemein eine Reaktion des Organismus und seiner Gewebe gegen verschiedenartige schädigende Reize verstanden. Unter schädigenden Reizen sind exogene und auch endogene Reize, wie z. B. Gewebsverletzungen, das Eindringen von Fremdkörpern, chemische Stoffe, bakterielle Toxine, Allergene, Immunkomplexe, Mikroorganismen, krankhafte Stoffwechselprodukte sowie Zerfallsprodukte von Geschwülsten zu verstehen Eng mit dem Entzündungsgeschehen verbunden sind die klassischen Symptome Schmerz und Fieber.

Es ist seit längerem bekannt, daß bestimmte körpereigene Substanzen, die sogenannten Mediatoren, eng mit dem Entzündungsablauf verbunden sind. Diese Mediatoren, denen eine zußerordentlich große pathogenetische Bedeutung zukommt, werden durch das schädigende Ereignis (Noxe) aus den Körperzellen freigesetzt. Als wichtigste und bekannteste Mediatoren gelten Histamin, 5-HT (5-Hydroxytryptamin), Bradykinin, die Prostaglandine, Prostacycline, die Leukotriene, Thromboxane und der erst in neuerer Zeit charakterisierte Thrombozyten aktivierende Faktor PAF (engl.: platelet activating factor).

Diese und weitere, im einzelnen nicht näher spezifizierte, Mediatoren wirken außerordentlich stark auf die Kontraktion der glatten Muskulatur, führen zur Herzfunktionsstörungen und beeinträchtigen die Integrität von Blutgefäßen und Schleimhäuten, wie z. B. die des Bronchialsystems. Sie bewirken außerdem die Aggregation von Thrombozyten und polymorphkernigen Leukozyten mit den schwerwiegenden Folgen einer anaphylaktischen Atemwegsverengung, Blutdruckabfall, Herz-Arrythmien, Plasmaexudation, Gewebsödeme, Hömokonzentration, Thrombozytopenie, Leukozytopenie, Verklumpung der Thrombozyten und polymorphkernigen Leukozyten in den Lungenkapillaren sowie schwerste Atmungsstörungen und Kreislaufkollaps.

Aufgrund ihres breiten pharmakologischen Wirkungsspektrums, ihrer großen Verbreitung im Organismus, ihrer Bildung durch zahlreiche physikalische, chemische, pathologische, pathophysiologische und pharmakologische Einflüsse sowie aufgrund ihrer Beteiligung bei einer Vielzahl pathophysiologischer Abläufe sind die Mediatoren bzw. deren pharmakologische Beeinflussung von größter medizinischer Bedeutung (vgl. "The Pharmacological Basis of Therapeutics, Ed. Goodman and Gilman, 6. Auflage, 1980, McMillan Publishing Company).

Dem PAF-Faktor scheint in der Pathogenese entzündlicher und allergischer Vorgänge eine besondere Bedeutung zuzukommen.

PAF ist ein Glycerophosphocholin mit der chemischen Bezeichnung 1-0-Alkyl-2-acetyl-sn-glyceryl-3-phosphorylcholin. Dieser Faktor wird von einer Reihe von Zellen, wie z. B. Makrophagen, basophilen und neutrophilen Granulozyten u. a. bei Aktivierung freigesetzt. Die Freisetzung von PAF führt in weiterer Folge zu den vorstehend beschriebenen pathologischen Zuständen und wahrscheinlich noch zu einer Reihe weiterer, bislang nicht völlig verstandener, pathologischer Symptome. PAF muß nicht direkt wirken, sondern kann seine Wirkung vielmehr über eine Stimulierung weiterer Mediatoren entfalten. Neuere Untersuchungen zeigte, daß PAF insbesondere eine wichtige Rolle in der Genese des klinischen Bronchialasthmas sowie bei anderen pathologischen Zuständen der Lunge, z. B. der obstruktiven Bronchitis besitzt.

Neben seiner wichtigen Rolle bei der Genese des Bronchialasthmas und bei der Anaphylaxie ist PAF als hochpotenter Entzündungsmediator mit den bereits oben beschriebenen pathologischen Wirkungen anzusehen.

Eine Vielzahl der oben genannten Mediatoren, einschließlich des PAF, werden durch eine membrangebundene Phospholipase aus Phospholipiden der Zellmembran freigesetzt, wobei einerseits Arachidonsäure und andererseits eine PAF-Vorstufe gebildet wird.

Ausgehend von Arachidonsäure werden zwei Gruppen von Mediatoren gebildet:

(i) durch das Enzym Cyclooxygenase die Prostaglandine einschließlich des Prostacyclins und des Thromboxans,

(ii) durch das Enyzm Lipoxygenase die offenkettigen Hydroperoxy- und Hydroxysäuren und insbesondere die Leukotriene.

Die PAF-Vorstufe wird durch eine Acetyltransferase in die aktive Verbindung überführt.

Pharmakologisch bedeutsam bei der Behandlung von Entzündungen sind zwei Gruppen von Wirksubstanzen; es handelt sich einmal um die sogenannten nicht-steroidalen Antiphlogistika, das sind Verbindungen und Derivate der Salicylsäure. Weitere Verbindungen mit bekannter antiphlogistischer Wirkung sind die Pyrazolonderivate, die para-Aminophenolderivate, die Indolderivate (z. B. Indomethacin) und Derivate der Propionsäure.

Die pharmakologische Wirkung aller dieser Verbindungen beruht darauf, daß sie die Cyclooxygenase zu hemmen vermögen und damit die Synthese der Prostaglandine oder Thromboxane unterbinden.

Salicylsäure bzw. ihre Derivate und die weiteren Verbindungen der gesamten Klasse sind mit einer Reihe schwerer und schwerster Nebenwirkungen behaftet. So führt z. B. die längerdauernde Verabreichung von Salicylsäurederivaten zur Magen- und Darmulzeration. Ebenfalls bekannt sind die relative Unverträglichkeit der Pyrazolonderivate, die hepatotoxische Wirkung der para-Aminophenolderivate, die allgemeine Unverträglichkeit von Indomethacin sowie die ulzerative Wirkung der Propionsäurederivate.

Ein weiterer ebenfalls schwerwiegender Nachteil der nicht-steroidalen Antiphlogistika besteht darin, daß sie unter Umständen die pathologische Wirkung der Mediatoren verstärken, indem durch die Hemmung der Cyclooxygenase vermehrt Substrat für die Lipoxygenase und damit für die Bildung von Leukotrienen bereitgestellt wird (Substratshift)

Den nicht-steroidalen Antiphlogistika gegenüber stehen die steroidalen Antiphlogistika, das sind die Corticosteroide und ihre Derivate. Die antiphlogistische Wirkung der Corticosteroide beruht auf ihrer Fähigkeit, sowohl die Phospholipase als auch die Lipoxygenase zu hemmen, wodurch der gesamte Arachldonsäurestoffwechsel inhibiert wird. Nachteilig für die Therapie mit Corticosteroiden sind die fatalen Nebenwirkungen, von denen nur beispielhaft die folgenden erwähnt werden sollen: Ulcera duodeni oder ventriculi, Myopathie, Osteoporosen, psychische Störungen, erhöhte Infektionsanfälligkeit, subkapsuläre Katarakte und dergleichen mehr.

Neben den Corticosteroiden sind selektive Lipoxygenasehemmer, wie z. B. Benoxaprofen, in Benutzung. Auch diese Substanzklasse ist mit schweren Nebenwirkungen behaftet, wie z. B. tödlich verlaufende exfoliative Dermatitiden (Syndrom der verbrühten Haut).

Aus der WO 90/04968 ist die Verwendung bestimmter Tanninderivate, insbesondere verschiedener Galloylchinasäuren, als Inhibitoren des HIV in Zeilen bekannt.

Biomed. Biochim. Acta, Baud 45, (1986), Nr. 10, Seiten 1267-1275 beschreibt die lipoxygenasehemmenden Eigenschaften von Propglgallat und weist auf die potentielle Verwendung zur Behandlung von Bronchialasthma und möglichenweise Entzündungen hin.

Die Anmelderin hat nun überraschend gefunden, daß bestimmte Gallussäurederivate, die aus der Pflanze Galphimia glauca isoliert werden können entweder als Einzelverbindung oder in jeder Kombination ausgezeichnete antiphlogistische Wirkungen besitzen. Insbesondere sind die Verbindungen zur Prophylaxe und Therapie des allergischen und des PAF-induzierten Bronchialasthmas geeignet.

Gegenstand der Erfindung ist daher die Verwendung von mindestens einer Verbindung ausgewählt aus Verbindungen der allgemeinen Formel:

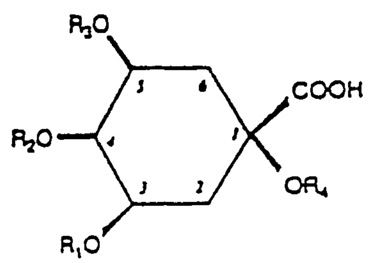

| | $R_1$ | $R_2$ | $R_3$ | $R_4$ |
|---|---|---|---|---|
| 1 | galloyl | galloyl | galloyl | H |
| 2 | galloyl | digalloyl | galloyl | H |
| 3 | galloyl | galloyl | digalloyl | H |
| 4 | digalloyl | galloyl | galloyl | H |
| 5 | galloyl | galloyl | galloyl | galloyl |

wobei Galloyl den Rest

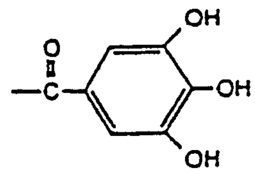

bedeutet und Digalloyl den Rest

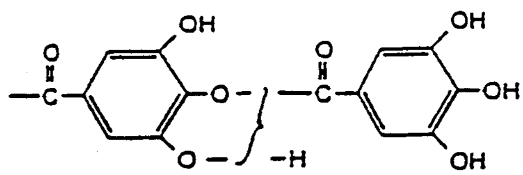

bedeutet,
Methylgallat und Gallussäure und den pharmazeutisch annehmbaren Salzen, Ether und Ester entweder allein oder in jeder Kombination zur Herstellung einer pharmazeutischen Zubereitung zur Behandlung oder Prophylaxe entzündlicher Erkrankungen.

Die allgemeine Formel umfaßt mehrere stereoisomere Verbindungen, die sich in der Stellung der Gruppen $R_4O$, $R_2O$ und $R_3O$ unterscheiden.

Zur erfindungsgemäßen Verwendung und von der allgemeinen Formel umfaßt sind alle isomeren Verbindungen.

Die Verbindungen der allgemeinen Formel sind aus Journal of Natural Products Vol. 52, No. 4, 762 - 768 (1989) bekannt. Sie besitzen eine Hemmwirkung auf die HIV Reverse Transkriptase und DNA Polymerase in vitro.

Die Verbindungen Methylgallat und Gallussäure sind ebenfalls bekannte Verbindungen (z. B. Merck Index, 9th Ed, 1986).

Eine insbesondere bevorzugt verwendete Verbindung ist die Verbindung Tetragalloylchinasäure, in der die Reste $R_1$ bis $R_4$ Galloyl bedeuten.

Am wirksamsten hinsichtlich der asthmaprotektiven Wirkung ist Tetragalloylchinasäure. Ebenfalls hochwirksam ist eine Kombination aller vorhin genannten Verbindungen.

Die vorstehend genannten Verbindungen können aus Galphimia glauca isoliert werden.

Unter dem Begriff "pharmazeutische Zubereitungen" sind auch pflanzliche Extrakte auf wäßriger oder nichtwäßriger Basis oder auf Grundlage eines pharmazeutisch annehmbaren Lösungsmittels, gegebenenfalls in Verbindung mit weiteren auf dem Gebiet der pharmazeutischen Technologie bekannten Träger-, Verdünnungsmittel und Additive zu verstehen, die mindestens eine der vorhin genannten Verbindungen allein oder in jeder beliebigen Kombination enthalten können.

Ein derartiger Extrakt enthält z. B. in wäßriger Basis im wesentlichen als Wirkstoffe Gallussäure, Gallussäuremethylester und Tetragalloylchinasäure in einem Mengenverhältnis von etwa 2,5 : 2,5 : 10.

Die unter der erfindungsgemäßen Verwendung hergestellten pharmazeutischen Zubereitungen besitzen die wesentliche und wichtige Eigenschaft, entzündliche Vorgänge allergischer Pathogenese, insbesondere PAF-induzierte Effekte, wie z.B. obstruktive Bronchitis, Asthmabronchiale, entzündliche Lungenerkrankungen, angiologische Störungen, entzündliche Gefäßprozesse, entzündliche Hauterkrankungen u.a., zu blokkieren.

Die pharmazeutischen Zubereitungen besitzen daher die Eigenschaft von steroidalen Antiphlogistika, ohne jedoch deren fatale Nebenwirkungen aufzuweisen und sind daher insgesamt wertvoll zur Prophylaxe und Behandlung von Entzündungsvorgängen im weitesten Sinn, an deren Entstehung die bekannten Mediatoren, wie z. B. Prostaglandine, Histamin, PAF, Leukotriene und Thromboxane eine Rolle spielen.

Im Gegensatz zu den nicht-steroidalen Antiphlogistika, die nicht gegen die von Leukozyten ausgelösten Effekte von chronisch entzündlichen Erkrankungen wirken, da sie die Leukotrien-Bildung nicht inhibieren, sind die hier verwendeten Verbindungen aufgrund ihrer überraschenden, ausgezeichneten und vorteilhaften Wirkung auch in dieser Hinsicht den bekannten Verbindungen überlegen.

Die erfindungsgemäß verwendeten Verbindungen erfüllen das Erfordernis für nicht-steroidale Antiphlogistika, die die Synthese der Leukotriene und Prostaglandine und Thromboxane inhibieren und den Effekten

des PAF-Faktors entgegenwirken. Sie sind frei von den mit den steroidalen oder nicht steroidalen Antiphlogistika verbundenen Nachteilen.

Die hier genannten pharmazeutischen Zubereitungen besitzen bislang keine feststellbare Toxizität.

Die Zubereitungen können in niedrigen Dosen zur Erzielung einer therapeutischen entzündungshemmenden Wirkung verabreicht werden.

Die pharmazeutischen Zubereitungen können zur Behandlung von Entzündungen der Gelenke, der Haut, der Schleimhäute sowie innerer Organe verwendet werden, unabhängig davon, ob die Entzündung durch Infektionserreger, immunologische Vorgänge oder Traumen hervorgerufen wurde. Hierbei sind insbesondere Entzündungsvorgänge des Bronchialsystems, wie Asthmabronchiale oder obstruktive Bronchitis besonders vorteilhaft zu behandelnde Indikationen. Weiterhin können die Arzneimittel zur Prophylaxe und Behandlung von Gefäß- und Herzerkrankungen verwendet werden, bei denen es wünschenswert erscheint, die Biosynthese von Entzündungsstoffen durch Thrombozyten zu inhibieren. Besonders vorteilhaft können die Arzneimittel zur Prophylaxe oder Behandlungen aller PAF und/oder Leukotrien induzierten Phänomene und insbesondere zur Behandlung des Bronchialraums verwendet werden.

Die erforderliche Menge der zu verwendenden Verbindung (im folgenden als der aktive Bestandteil bezeichnet) im Arzneimittel für die gewünschte therapeutische Wirkung hängt von der jeweiligen Verbindung, der Verabreichungsart und von dem zu behandelnden Subjekt, sowie der jeweiligen Krankheit ab. Eine geeignete Dosis einer Verbindung zur Verabreichung an einen Säuger, der an einer Entzündung, einem schmerzhaften oder fiebrigen Zustand, wie vorhin beschrieben, leidet, beträgt etwa 0,1 $\mu$g bis 500 mg des aktiven Bestandteils pro Kilogramm Körpergewicht. Im Falle einer systemischen Verabreichung kann die Dosis im Bereich von 0,5 bis 500 mg der aktiven Verbindung pro Kilogramm Körpergewicht, und die am meisten bevorzugte Dosis im Bereich von 0,5 bis 50 mg pro Kilogramm Körpergewicht, z. B. 5 bis 25 mg pro Kilgramm Körpergewicht, betragen, die gegebenenfalls mehrmals täglich, insbesondere zwei- oder dreimal täglich verabreicht wird.

Im Falle einer topischen Verabreichung. z. B. auf die Haut oder Schleimhäute, kann die geeignete Dosis deutlich höher liegen.

Obwohl es grundsätzlich möglich ist, den oder die aktiven Bestandteil(e) alleine zu verabreichen, ist es vorzuziehen, daß der aktive Bestandteil in Form einer pharmazeutischen Formulierung, die eine Verbindung wie vorstehend beschrieben und einen dafür pharmazeutisch annehmbaren Trägerstoff enthält, verabreicht wird. Üblicherweise liegt der aktive Bestandteil in einer derartigen Formulierung in einer Konzentration von 0,1 bis 99,9 Gew.-% der Formulierung vor. Üblicherweise enthält eine Einzeldosis einer Formulierung zwischen 0,1 mg und 1 g des aktiven Bestandteils. Für die topische Verabreichung beträgt die Konzentration des aktiven Bestandteils vorzugsweise 1 bis 2 Gew.-% der Zubereitung, jedoch kann der aktive Bestandteil bis zu 10 Gew.-% ausmachen. Zubereitungen, die für eine nasale oder buccale Verabreichung vorgesehen sind, wie z. B. selbstzerstäubende Pulver, Sprays oder andere bekannte und übliche Vorrichtungen können 0,1 bis 20 Gew.-%, z. B. 2 Gew.-%, des aktiven Bestandteils enthalten.

Die pharmazeutischen Zubereitungen zur Verwendung in der Veterinär- als auch in der Humanmedizin enthalten den aktiven Bestandteil in Verbindung mit einem dafür pharmazeutisch annehmbaren Trägerstoff und gegebenenfalls weiteren therapeutisch aktiven Bestandteilen. Der Trägerstoff muß annehmbar in dem Sinne sein, daß er mit den anderen Bestandteilen der Formulierung kompatibel ist und keine nachteilige Wirkung auf den Empfänger der Formulierung besitzt.

Die Formulierungen liegen geeigneterweise in Form einer oralen, ophtalmologischen, rektalen, parenteralen (einschließlich subkutanen, intramuskulären, und intravenösen) intraartikulären, topischen, nasalen oder buccalen Verabreichungsform vor.

Die Formulierungen liegen üblicherweise in Form einer Einzeldosis vor und können durch jedes der auf dem Gebiet der pharmazeutischen Technologie bekannten Verfahren hergestellt werden. Im wesentlichen enthalten alle Verfahren den Schritt des Zusammenbringens des aktiven Bestandteils mit dem Trägerstoff, gegebenenfalls mit einem oder mehreren zusätzlichen Bestandteilen. Im allgemeinen werden die Formulierungen durch gleichmäßiges und inniges Vermischen des aktiven Bestandteils mit einem flüssigen Träger oder einem fein verteilten festen Träger oder beiden und anschließend, falls erforderlich, Formen des Produktes in die gewünschte Zubereitungsform, hergestellt.

Die Formulierungen für die orale Verabreichung können in Form von diskreten Einheiten, wie z. B. Kapseln, Cachets, Tabletten oder Pastilien, vorliegen, wobei jede Form eine bestimmte Menge des aktiven Bestandteils enthält. Sie können ebenfalls in Form eines Pulvers oder in Form eines Granulats oder in Form einer Lösung oder einer Suspension in einer wäßrigen oder nicht-wäßrigen Flüssigkeit, oder in Form einer Öl-in-Wasser-Emulsion oder Wasser-in-Öl-Emulsion, vorliegen. Der aktive Bestandteil kann ebenfalls in Form eines Bolus, eines Elektuariums oder einer Paste vorliegen.

Wenn die Zubereitungen in Form einer Tablette vorliegen, kann man diese durch Verpressen oder Vergießen des aktiven Bestandteils gegebenenfalls zusammen mit einem oder mehreren zusätzlichen Bestandteilen herstellen. Verpreßte Tabletten kann man durch Pressen des aktiven Bestandteils in freifließender Form, z. B. als Pulver oder als Granulat, gegebenenfalls vermischt mit einem Bindemittel, einem Gleitmittel, einem inerten Verdünnungsmittel, oberflächenaktiven oder Dispersions-Mittel in einer geeigneten Vorrichtung herstellen. Gegossene Tabletten kann man durch Vergießen einer Mischung des in Pulverform vorliegenden aktiven Bestandteils und eines geeigneten Trägerstoffs, der mit einem inerten flüssigen Verdünnungsmittel befeuchtet ist, in einer geeigneten Vorrichtung herstellen.

Die Zubereitungen für die rektale Verabreichung können in Form von Suppositorien vorliegen, wobei der aktive Bestandteil in einem Träger, z. B. aus Kakaobutter, enthalten ist. Sie können auch in Form eines Klistiers vorliegen.

Wenn die Formulierungen für die parenterale Verabreichung vorgesehen sind, enthalten sie üblicherweise eine sterile wäßrige Zubereitung des aktiven Bestandteils, die vorzugweise mit dem Blut des Empfängers isotonisch ist.

Zubereitungen, die für die intraartikuläre Verabreichung geeignet sind, können in Form einer sterilen wäßrigen Zubereitung des aktiven Bestandteils vorliegen, wobei der aktive Bestandteil gegebenenfalls in mikrokristalliner Form vorliegt, z. B. in Form einer wäßrigen mikrokristallinen Suspension.

Die Formulierungen können gleichfalls in Form einer liposomalen Zubereitung oder in Form eines bioabbaubaren Polymersystems zur Verabreichung des aktiven Bestandteils vorliegen.

Für die topische Verabreichung geeignete Formulierungen enthalten flüssige oder halbflüssige Zubereitungen, wie z. B. Einreibemittel, Lotionen, Verbände, Öl-in-Wasser- oder Wasser-in-Öl-Emulsionen, wie z. B. Cremes, Salben oder Pasten, oder Lösungen oder Suspensionen, wie z. B. Tropfen. Zum Beispiel kann der aktive Bestandteil für die ophthalmologische Verabreichung in Form von wäßrigen Augentropfen, beispielsweise in Form einer 0,1 bis 1,0 %igen Lösung, vorliegen.

Für die Verabreichung durch die Nase oder in die Mundhöhle geeignete Formulierungen liegen in Form eines selbstzerstäubenden Pulvers oder in Form von Sprayzubereitungen, z. B. als Aerosol, vor. Die Formulierungen ergeben nach Dispersion vorzugsweise eine Teilchengröße im Bereich von 1 bis 200 µm.

Formulierungen können den aktiven Bestandteil auch in einer wäßrigen oder verdünnten alkoholischen Lösung enthalten. Der aktive Bestandteil kann gegebenenfalls durch ein Sprühgerät in einen feinen Nebel überführt werden, der von den Patienten inhaliert wird.

Derartige pharmazeutische Zubereitungen enthalten üblicherweise einen Geschmacksstoff, wie z. B. Natriumsaccharin und ein ätherisches Öl. Ebenfalls kann eine Puffersubstanz und/oder ein oberflächenaktives Mittel in derartigen Zubereitungen zusammen mit Konservierungsmitteln, wie z. B. Methylhydroxybenzoat, enthalten sein.

Andere Zubereitungen, die zur Verabreichung durch die Nase geeignet sind, bestehen aus einem groben Pulver, das eine Teilchengröße von 20 bis 500 µm aufweist, das auf die gleiche Art und Weise wie Schnupftabak verabreicht wird.

Zusätzlich zu den vorhin genannten Bestandteilen können die Formulierungen einen oder mehrere weitere übliche und bekannte Komponenten, wie z. B. Verdünnungsmittel, Puffersubstanzen, Geschmacksstoffe, Bindemittel, oberflächenaktive Mittel, Verdickungsmittel, Gleitmittel, Konservierungsmittel, Antioxidantien, Emulgatoren und dergleichen enthalten.

Die folgenden Beispiel erläutern die Erfindung, ohne diese auf die Beispiele zu beschränken.

<u>Beispiel 1</u> (nicht zur Erfindung gehörend)

Isolierung eines Extraktes aus Galphimia glauca

Galphimia glauca (Thyrallis glauca) ist eine im mittelamerikanischen Raum beheimatete Pflanze aus der Familie der Malpighiacaen.

Zur Isolierung der vorstehend beschriebenen Verbindungen wird von der getrockneten Pflanze ausgegangen, die zunächst mit Alkanolen mit 1 bis 3 Kohlenstoffatomen wie Methanol, Ethanol und/oder Propanol, insbesondere mit Methanol, erschöpfend unter Verwendung bekannter Verfahren extrahiert wird. Dies kann durch Soxhlet-Extraktion, Perkolation, oder Maceration geschehen. Ebenfalls können überkritische Gase (z. B. $CO_2$ oder Butan) nach an sich bekannten Verfahren zur Extraktion verwendet werden (Fraktion I). Nach Entfernung mindestens eines Teils, bevorzugt des gesamten Extraktionsmittels, werden von dem Extrakt die lipophileren Anteile abgetrennt, indem der Extrakt mit Wasser aufgenommen und das Gemisch mit chlorierten Kohlenwasserstoffen mit einem Siedepunkt von maximal 120 °C, insbesondere mit Chloroform und/oder Methylenchlorid extrahiert wird (Fraktion III). Die zurückbleibende wäßrige Phase wird

mit Estern der Essigsäure mit Alkanolen mit 1 bis 4 Kohlenstoffatomen, z. B. Methyl-, Ethyl-, Propyl- und/oder Butylacetat, insbesondere mit Ethylacetat, extrahiert; dabei geht in die Essigesterphase eine intermediär polare Fraktion über, die isoliert wird (Fraktion II). Aus dieser Fraktion werden die hydrophileren Bestandteile abgetrennt und zwar insbesondere mittels Flash-Chromatographie mit Kieselgel als stationärer Phase mit Methylenchlorid/Aceton/Methanol/Ameisensäure als Elutionsmittel; ein typisches Elutionsmittel enthält 90 % Methylenchlorid, 10 % Aceton und 10 % Metahnol, dem 1 % Ameisensäure zugesetzt wird. Die zuerst übergehende Fraktion wird gegebenenfalls weiter aufgearbeitet (Extrakt); die anschließend erscheinende, hydrophilere Fraktion enthält ausschließlich Flavonglykoside und ist pharmakologisch unwirksam.

Die bei der vorstehend beschriebenen Chromatographie erhaltene lipophilere Fraktion enthält gemäß dem Dünnschichtchromatogramm überwiegend Gallussäure, Gallussäuremethylester, Quercetin und einige Nebenprodukte. Zur Abtrennung der in ihr enthaltenen Ameisensäure wird die Fraktion mit ca. 20 ml destilliertem Wasser versetzt und am Rotationsverdampfer schonend vom organischen Lösungsmittel befreit. Die wäßrig-ameisensaure Lösung wurde anschließend auf eine mit RP-Material gepackte und mit Wasser equilibrierte Säule gegeben. Die Ameisensäure wurde durch Wasser eluiert und die gesuchten Wirkstoffe blieben am Anfang der Säule haften; sie wurden mit 70 % Methanol von der Säule eluiert und das verbleibende Methanol wurde abgedampft. Die zurückbleibenden wäßrigen Lösungen wurden lyophilisiert.

Bei der vorstehend erläuterten Dünnschichtchromatographie bzw. HPLC-Chromatographie kamen die folgenden Materialien und Methoden zur Anwendung.

**(a) Dünnschichtchromatographie:**
Adsorbentien:
Kieselgel 60 F 254-Fertigplatten 20 x 20 cm;
Schichtdicke: 0,25 cm;
Laufmittelsystem DC-G 1:
Toluol-Aceton-Ameisensäure (55 : 30 : 4,5);
Detektionsmittel:
Det-1 (Phenole): $FeCl_3$ (5 % in 0,1 n HCl);
Det-2 (Flavonoide und Pflanzensäuren): Naturstoffreagenz nach NEU (1 % in Methanol) mit PEG-400-Lösung (5 % in Ethanol) nachbesprüht;

**(b) HPLC:**
Adsorbentien:
Säule: Hibar 125-4 Lichrospher 100 CH-18/2 (5 µm);
Trennsysteme:
HPLC-G 1: Gradient: 4 - 20 % in 30 min., linearer Anstieg:
HPLC-G 2: Gradient: 4 - 30 % in 30 min., 30 - 70 % in 10 min., linearer Anstieg;
Fließmittel:
A: Wasser;
B: Acetonitril;
jeweils unter Zusatz von 10 ml 0,1 n Phosphorsäure/l;
Detektion:
jeweils bei 265 nm.

**Isolierung der Tetragalloylchinasäure**

Zur Isolierung der Tetragalloylchinasäure wird die Fraktion II zuerst durch Zusatz von HCl (16 % HCl; 15 min., 60 °C) hydrolysiert. Das Gemisch wird anschließend über eine Silicagelsäule unter Verwendung eines Lösungsmittelsystems aus Ethylacetat/Methanol/ Wasser/Ameisensäure (85 : 10 : 3 : 0,5) und durch präparative HPLC über RP-Material unter Verwendung von Acetonitril/Wasser als Lösungsmittelsystem isoliert. Die isolierte Verbindung wurde durch $^1H$-NMR- und $^{13}C$-NMR-Analysen als Tetragalloylchinasäure identifiziert und charakterisiert.

**Beispiel 2**

**Biologische Untersuchungen**

Männliche weiße Meerschweinchen wurden durch intraperitoneale und intramuskuläre Injektion von Ovalbumin nach bekannten Verfahren sensibilisiert [1]. Inhalationsreizversuche wurden 4 bis 6 Wochen

später durchgeführt.

Alle Versuche wurden nach einem Zufallsprotokoll [2] durchgeführt:

Gruppen aus 10 bis 14 Tieren wurden in zwei Untergruppen aufgeteilt und entweder mit dem zu untersuchenden Material oder mit Kontrollösungen behandelt. Nach einer Woche wurden die Tiere ein zweites Mal behandelt, wobei jene, die mit der Kontrolle behandelt worden waren, die aktive Verbindung und umgekehrt erhielten (jedes Tier erhielt einmal den Pflanzenextrakt, die zu testende Fraktion oder Verbindung oder das entsprechende Lösungsmittel).

1 Stunde, 12 Stunden, 4 oder 7 Tage nach der Vorbehandlung wurden die Tiere verschiedenen Inhalationsreizen ausgesetzt. Ovalbumin, Histaminhydrochlorid und Acetylcholin wurden in physiologischer Kochsalzlösung (1 : 99, 1 : 999, 1 : 99 w/v), PAF zuerst in Ethanol und anschließend in Kochsalzlösung, die 0,25 % BSA enthielt, zu einer Endkonzentration von 1 µg PAF/ml gelöst. Ovalbumin, PAF, Histamin und Acetylcholin wurden als Aerosol, das ultraschallvernebelt wurde, verabreicht.

Spontan atmende Tiere wurden in einen Zwei-Kammer-Körper-Plethysmograph, in dem beide Kammern durch einen wassergefüllten Gummikragen um den Kopf der Tiere getrennt waren, gegeben. Volumenänderungen in beiden Kammern wurden durch Drücküberträgersysteme gemessen. Der Grad der bronchialen Obstruktion wurde mittels des Parameters "compressed air" bestimmt. Diese Technik ist etwa zehnmal sensititver als andere invasive Methoden (Dorsch et al., Pflügers Arch., 1981, 391: 236 bis 241).

Die Wirkung der Pflanzenextrakte oder der Verbindungen auf die PAF-induzierte Reaktion wurde durch die aufeinanderfolgende Inhalierung von Histamin, PAF und Histamin untersucht:

Eine Gruppe von Tieren wurde in zwei Untergruppen getrennt und beide Untergruppen inhalierten Histamin in einer Konzentration, die eine mittlere bronchiale Obstruktion auslöst. Eine Stunde später erhielt eine Gruppe die zu untersuchenden Substanzen, die andere Gruppe erhielt das Lösungsmittel allein. Beide Gruppen wurden 12 Stunden später durch die aufeinanderfolgende Inhalierung von PAF und Histamin einem Reiz unterworfen, wobei zuerst 1 µg PAF und 60 Minuten später die gleiche Histamindosis wie zuvor verabreicht wurde. Der zweite Histaminreiz führt in Kontrolltieren üblicherweise zu asthmatischen Reaktionen, die dreimal so stark sind als beim ersten Mal (Dorsch et al., Int. Arch. Allergy Appl. Immunol., 88: 228 bis 230 (1988)).

Die Ergebnisse dieser Versuche sind in Tabelle 1 dargestellt. Die in der Tabelle dargestellten Ergebnisse zeigen, daß die Fraktion I und die Fraktion III keine signifikante Aktivität besitzen. Demgegenüber ist die Fraktion II in der Lage, bei einer verabreichten Konzentration von 30 mg/kg sowohl das allergische als auch das PAF-induzierte Bronchialasthma zu mehr als 50 % zu inhibieren. Die Fraktion II enthält Gallussäure, Gallussäuremethylester, Tetragalloylchinasäure und Quercetin. Die gleichen Ergebnisse zeigt die Subfraktion II/1, die ebenfalls bei einer Verabreichungskonzentration von 30 mg/kg sowohl das allergische als auch das PAF-induzierte Bronchialasthma zu mehr als 50 % zu inhibieren vermag. Die Einzelverbindungen Methylgallat, Gallussäure und Quercetin zeigen in etwa die gleiche Aktivität bei der Verhinderung der Bronchialobstruktion nach einer Allergen-Inhalation und für den Fall der PAF-induzierten Bronchialobstruktion zeigt das Methylgallat etwas höhere Aktivität als die Mischung aus Quercetin, Gallussäure und Protocatechusäure. Die höchste Aktivität zeigte die Verbindung Tetragalloylchinasäure, die bei der geringen oralen Dosis von 5 mg/kg die Bronchialreaktionen auf einen Allergenreiz um 86 ± 24 % und eine durch PAF induzierte bronchiale Reatkion um etwa 60 % inhibiert.

Literatur:

[1] Dorsch et al., Pflügers Arch. 391: 236 - 241 (1981)
[2] Dorsch et al. , Naunyn-Schmiedebergs Arch. Pharmacol. 325, 275 - 282 (1984)

Tabelle 1

| Fraktion/ Verbindung | Konzentration (mg/kg) | % Inhibierung der bronchialen Obstruktion nach Allergen-Inhalation 1. Reiz (%) | 2. Reiz (%) | PAF-Inhalation (%) |
|---|---|---|---|---|
| Fraktion II | 30 | 55 ± 52 | 54 ± 28 | 51 ± 32 |
| Fraktion I | 30 | | | 12 ± 35 |
| Fraktion III | 30 | 19 ± 68 | 22 ± 65 | 18 ± 24 |
| Subfraktion. II/1 | 30 | 59 ± 32 | 43 ± 46 | 57 ± 29 |
| Methylgallat | 45 | 43 ± 34 | 41 ± 24 | 65 ± 24 |
| Gallussäure | 45 | 37 ± 35 | 40 ± 31 | |
| Tetragalloyl-chinasäure | 5 | 86 ± 24 | 57 ± 79 | 60 ± 43 |
| Quercetin | 45 | 53 ± 23 | 34 ± 33 | |
| Mischung | 45 | | | 36 ± 45 |
| Fraktion II (3 Tage 3 x 2 mg/kg) | | 75 ± 63 | | 71 ± 38 |

**Patentansprüche**

1. Verwendung einer Verbindung ausgewählt aus Verbindungen der Formel

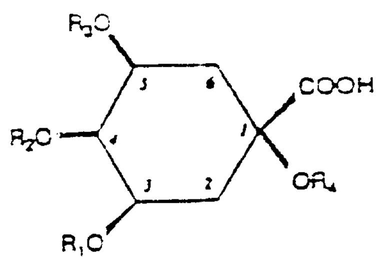

| | $R_1$ | $R_2$ | $R_3$ | $R_4$ |
|---|---|---|---|---|
| 1 | galloyl | galloyl | galloyl | H |
| 2 | galloyl | digalloyl | galloyl | H |
| 3 | galloyl | galloyl | digalloyl | H |
| 4 | digalloyl | galloyl | galloyl | H |
| 5 | galloyl | galloyl | galloyl | galloyl |

wobei Galloyl den Rest

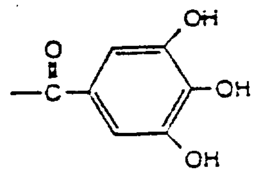

bedeutet und Digalloyl den Rest

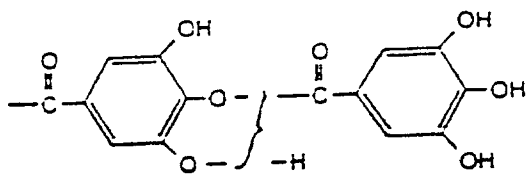

bedeutet
und den pharmazeutisch annehmbaren Salzen, Ether und Ester, Methylgallat und Gallussäure zusammen mit pharmazeutisch annehmbaren Träger- und Verdünnungsmitteln zur Herstellung einer pharmazeutischen Zubereitung zur Behandlung oder Prophylaxe entzündlicher Erkrankungen.

**2.** Verwendung nach Anspruch 1, dadurch **gekennzeichnet,** daß die Verbindungen Gallussäure, Gallussäure-Methylester und eine Verbindung der allgemeinen Formel, wie in Anspruch 1 definiert, sind.

**3.** Verwendung nach Anspruch 1, dadurch **gekennzeichnet,** daß die Verbindung Tetragalloylchinasäure ist.

**4.** Verwendung nach einem der Ansprüche 1 bis 3, zur Behandlung von Erkrankungen des rheumatischen Formenkreises, allergisch-entzündlicher und immunologisch-entzündlicher Erkrankungen, Asthmabronchiale, obstruktiver Bronchitis und entzündlicher Hauterkrankungen.

## Claims

**1.** Use of a compound selected from compounds of the formula

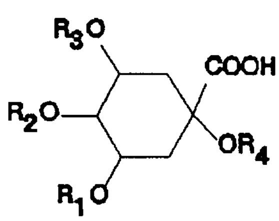

| | $R_1$ | $R_2$ | $R_3$ | $R_4$ |
|---|---|---|---|---|
| 1 | galloyl | galloyl | galloyl | H |
| 2 | galloyl | digalloyl | galloyl | H |
| 3 | galloyl | galloyl | digalloyl | H |
| 4 | digalloyl | galloyl | galloyl | H |
| 5 | galloyl | galloyl | galloyl | galloyl |

where galloyl means the group

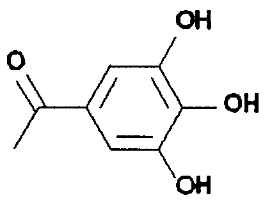

and digalloyl means the group

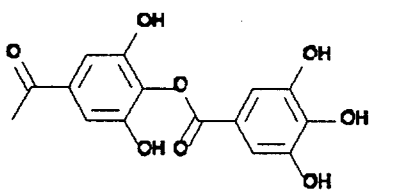

or

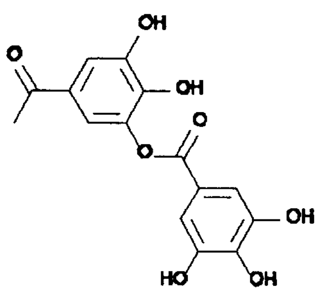

and the pharmaceutically acceptable salts, ethyl oxides and esters, methyl gallate and gallic acid together with pharmaceutically acceptable carriers and diluents for the manufacture of a pharmaceutical preparation for the treatment or prophylaxis of inflammatory diseases.

2. Use according to Claim 1 characterized in that the compounds are gallic acid, gallic acid methyl ester and a compound of the general formula as defined in Claim 1.

3. Use according to Claim 1 characterized in that the compound is tetragalloyl quinnic acid.

4. Use according to one of the Claims 1 to 3 for the treatment of diseases of the rheumatic complex, allergic inflammatory and immunological inflammatory diseases, bronchial asthma, obstructive bronchitis and inflammatory skin diseases.

**Revendications**

1. Utilisation d'un composé choisi parmi les composés de formule

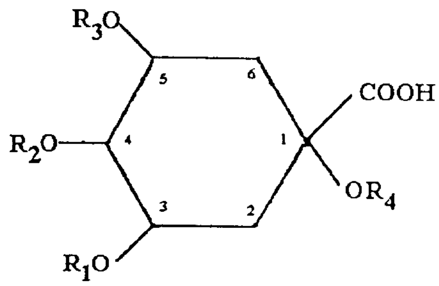

| | R1 | R2 | R3 | R4 |
|---|---|---|---|---|
| 1 | galloyle | galloyle | galloyle | H |
| 2 | galloyle | digalloyle | galloyle | H |
| 3 | galloyle | galloyle | digalloyle | H |
| 4 | digalloyle | galloyle | galloyle | H |
| 5 | galloyle | galloyle | galloyle | galloyle |

dans laquelle galloyle représente le résidu

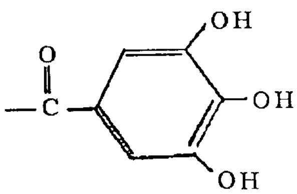

et digalloyle représente le résidu

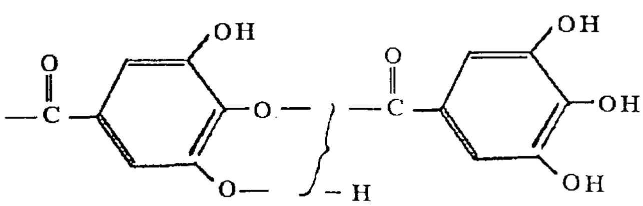

et les sels, éthers et esters pharmaceutiquement acceptables, le méthylgallate et l'acide gallique, avec des véhicules et des diluants pharmaceutiquement acceptables pour la production d'une préparation pharmaceutique pour le traitement curatif ou préventif de maladies inflammatoires.

2. Utilisation selon la revendication 1, caractérisée en ce que les composés sont l'acide gallique, l'ester méthylique d'acide gallique et un composé de formule générale telle que définie à la revendication 1.

3. Utilisation selon la revendication 1, caractérisée en ce que le composé est l'acide tétragalloylquinique.

4. Utilisation selon l'une des revendications 1 à 3, pour le traitement de maladies de type rhumatismal, de maladies inflammatoires allergiques et inflammatoires immunologiques, de l'asthme bronchique, de la bronchite obstructive et de maladies inflammatoires cutanées.